# EUROPEAN PATENT SPECIFICATION

(11) **EP 0 449 856 B1**
(45) Date of publication and mention of the grant of the patent: **12.09.2001**
(21) Application number: 90900035.8
(22) Date of filing: 15.12.1989
(51) Int. Cl.: C12N 15/31, C07K 14/315, A61K 39/09, C12N 5/10

(54) **PNEUMOLYSIN MUTANTS AND PNEUMOCOCCAL VACCINES MADE THEREFROM**
PNEUMOLYSIN-MUTANTEN UND PNEUMOKOKKEN-IMPFSTOFFE DARAUS
MUTANTS DE PNEUMOLYSINE ET VACCINS CONTRE LE PNEUMOCOQUE OBTENUS A PARTIR DE TELS MUTANTS

(30) Priority: 16.12.1988 AU 198988
(43) Date of publication of application: 09.10.1991
(73) Proprietor: DE STAAT DER NEDERLANDEN VERTEGENWOORDIGD DOOR DE MINISTER VAN WELZIJN, VOLKSGEZONDHEID EN CULTUUR, NL-2280 HK Rijswijk (NL)
(72) Inventor: PATON, James Cleland, Parkside, S.A. 5063 (AU); BOULNOIS, Graham John, Leicester, Leicestershire LE1 9HN (GB); ANDREW, Peter William, Leicester, Leicestershire LE1 9HN (GB); MITCHELL, Timothy John, Burton-on-Trent, Staffordshire DE12 7AA (GB); WALKER, John Arthur, Memphis, TN 38104 (US)
(74) Representative: de Bruijn, Leendert C.
(86) International application number: AU8900539
(87) International publication number: WO9006951

(56) References cited:
- GENE, vol. 77, no. 2, 30th April 1989, pages 211-218, Elsevier Science, Amsterdam, NL; S. TAIRA et al.: "Production of pneumolysin, a pneumococcal toxin, in Bacillus subtilis"
- INFECTION AND IMMUNITY, vol. 54, no. 1, October 1986, pages 50-55, American Society for Microbiology, Washington, DC, US; J.C. PATON et al.: "Cloning and expression in Escherichia coli of the Streptococcus pneumoniae gene encoding pneumolysin"
- Infection and Immunity, vol. 57(8), Aug 1989, p. 2547-2552, F.K. Saunders et al "Pneumolysin, the Thiol-Activated Toxin of Streptococcus pneumoniae, does not require a Thiol Group for In Vitro Activity"
- Infection and Immunity, vol. 55(5) May 1987, p. 1184-1189, Walker, J.A. et al "Molecular Cloning, Characterization, and complete Nucleotide Sequence of the Gene for Pneumolysin, the Sulfhydryl-Activated Toxin of Streptococcus pneumoniae
- Journal of Clinical Microbiology Feb 1987 p222-225 KRZYSZTOF KANCLERSKI et al "Production and Purification of Streptococcus pneumoniae Hemolysin (Pheumolysin).

## Description

This invention relates to mutants of the toxin pneumolysin and pneumococcal vaccines based on these mutants.

### BACKGROUND

*Streptococcus pneumoniae* (pneumococcus) is an important pathogen, causing invasive diseases such as pneumonia, meningitis and bacteraemia. Even in regions where effective antibiotic therapy is freely available, the mortality rate from pneumococcal pneumonia can be as high as 19% in hospitalized patients and this increases to 30-40% in patients with bacteraemia. These high mortality rates have been reported in the U.S.A. where pneumonia, of which *S. pneumoniae* is the commonest cause, is the fifth ranking cause of death. Indeed, pneumonia is the only infectious disease amongst the top ten causes of death in that country. In the United States mortality rates for pneumococcal meningitis range from 13-45%. In developing countries, in excess of 3 million children under the age of 5 years die each year from pneumonia, and again *S. pneumoniae* is the commonest causative agent. *S. pneumoniae* also causes less serious, but highly prevalent infections such as otitis media and sinusitis, which have a significant impact on health-care costs in developed countries. Otitis media is especially important in young children; sinusitis affects both children and adults.

In the late 1970's, a vaccine was licensed for the purpose of preventing serious infections, especially bacterial pneumonia and for protecting certain groups, such as splenectomized individuals and young children, who are particularly susceptible to fulminating pneumococcal disease. The vaccine is composed of purified capsular polysaccharides, which are the predominant pneumococcal surface antigens. However, each serotype of *S. pneumoniae* (of which there are 83) has a structurally distinct capsular polysaccharide, and immunization with one serotype confers no protection whatsoever against the vast majority of the others. The vaccine currently licensed in Australia contains polysaccharides purified from the 23 most common serotypes, which account for approximately 90% of pneumococcal infections in this country.

Protection even against those serotypes cntained in the vaccine is by no means complete, and there have been several reports of serious, even fatal infections occurring in vaccinated high-risk individuals. The efficacy of the vaccine is poorest in young children, and several studies, including one conducted in Adelaide, have shown that the existing formulation has little or no demonstrable clinical benefit in this group. This apparent failure of the vaccine appears to be related to the poor immunogenecity of certain pneumococcal polysaccharides in children under 5 years of age. We have shown that the antibody response is particularly poor to the five serotypes which most commonly cause disease in children (types 6, 14, 18, 19 and 23). Indeed, the antibody response to these pneumococcal polysaccharides only approaches adult levels in children over 8 years of age at the time of vaccination. (Vaccines, Ed. S.A.Plotkin and E.A.Mortimer, 2nd Ed. 1994, W.B.Saunders Company, ISBN 0-1726-6584-5, page 535 left column, last paragraph - right column, first paragraph).

In view of this, a vaccine, including antigens other than the capsular polysaccharides seems to be required to protect young children from pneumococcal infection. One such antigen could be pneumolysin, a protein toxin produced by all virulent *S.pneumoniae* isolates. Immunization of mice with this protein has been found to confer a degree of protection from pneumococcal infection (Vaccines, loc.cit., page 550, right column, first paragraph).

However, there is a difficulty in that pneumolysin is toxic to humans. Thus pneumolysin included in a vaccine must therefore be substantially non-toxic. However, the rendering of a pneumolysin non-toxic by most currently employed methods would be likely to alter the basic configuration of the protein so as to be immunogenically distinct from the native or wild-type pneumolysin. An immune response elicited by an altered protein that is immunogenically distinct from the native pneumolysin will have a decreased protective capacity or no protective capacity. In this respect it is pointed at Infection and Immunity, vol.54, no.1, 1986, pages 50-55, Paton J.C. et al. disclosing the pneumolysin mutants pJCP21 and pJCP22. However, said mutants do not provide a protective immune response

Thus the difficulty is to produce an altered pneumolysin that is non-toxic and at the same time sufficiently immunogenecally similar to the toxic form to elicit a protective immune response.

An altered pneumolysin with the above characteristics can then be used in a number of ways in a vaccine. Thus the altered pneumolysin may be used by itself to immunise, or alternatively the altered pneumolysin may be conjugated to pneumococcal polysaccharide, or alternatively may be included in a vaccine wherein pneumococcal polysaccharides may be conjugated to another protein and the altered pneumolysin is present in a non-conjugated form only. Alternatively, pneumococcal polysaccharide and pneumolysin may both be used in an unconjugated form.

### DESCRIPTION OF INVENTION

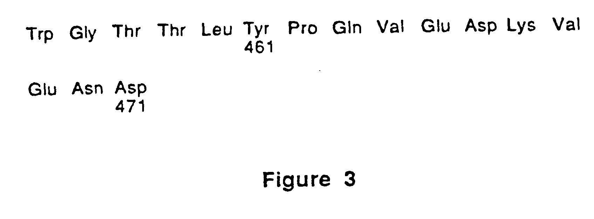

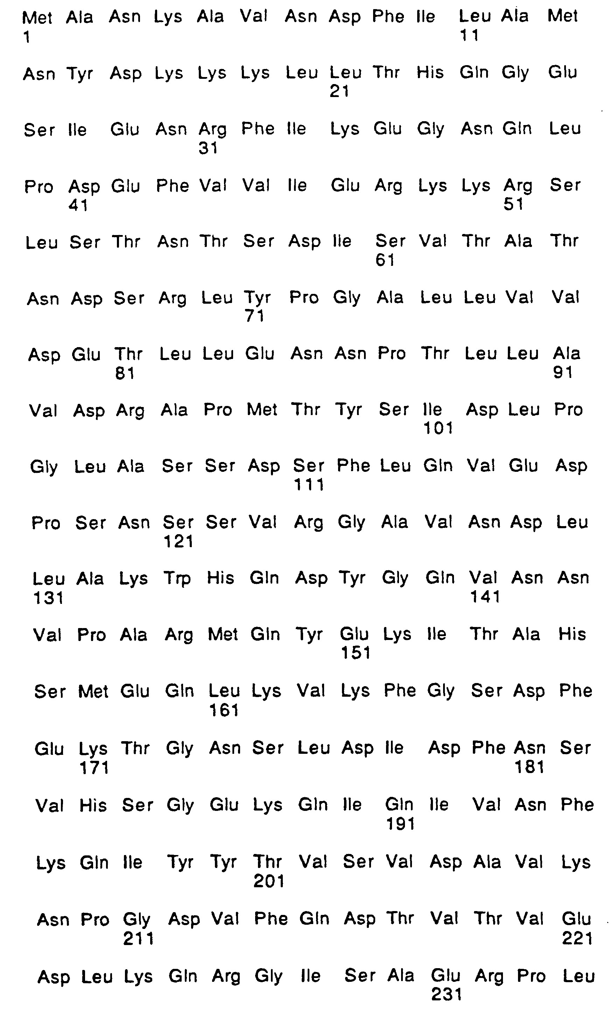

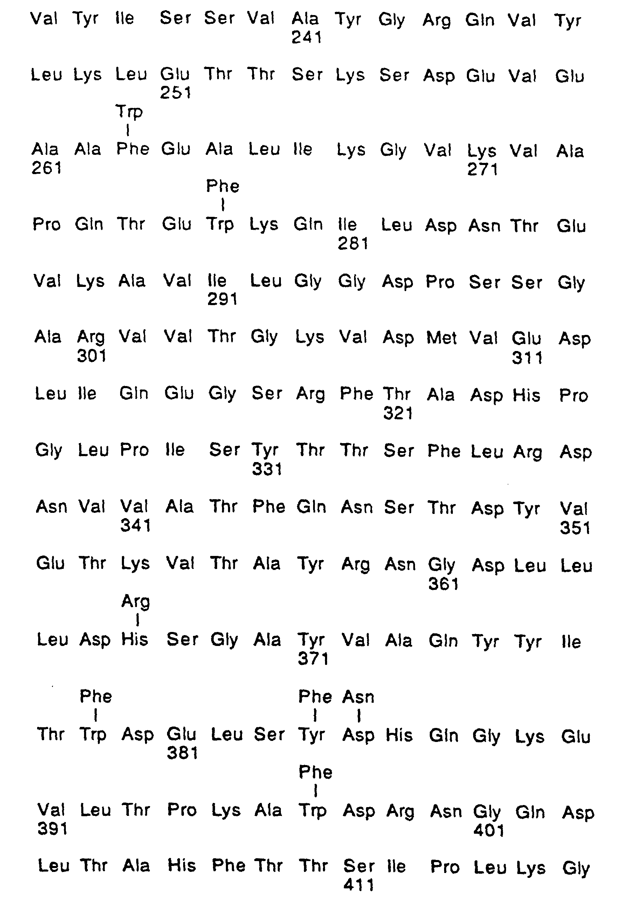

In a broad form therefore the invention may be said to reside in a mutant pneumolysin being substantially non-toxic and being capable of eliciting a protective immune response in an animal being reactive to wild-type pneumolysin, **characterized in that** the mutant pneumolysin has the amino acid sequence illustrated in Figure 3, which sequence has been altered by at least one amino acid substitution, deletion or blocking in positions 257 to 297 and/or positions 367 to 397 and/or positions 424-437.

Preferably the mutant pneumolysin has been altered in positions 367-397 and has reduced complement binding activity as compared to wild-type pneumolysin. Reduction in the complement binding activity results in less inflammation at the site of administering the vaccine.

Preferably the mutant pneumolysin has been altered in positions 257-297 and has reduced Fc binding activity as compared to wild-type pneumolysin. Reduction in the Fc binding activity results in less inflammation at the site of administering the vaccine.

Preferably the mutant pneumolysin is altered by reason of one or more amino acid substitutions relative to wild-type pneumolysin.

The pneumolysin may be altered in that the amino acid present at any one or more than one of residue sites 367, 384, 385, 428, 433 or 435 of wild-type pneumolysin are replaced, removed or blocked.

In a further form the invention could be said to reside in a vaccine including an altered pneumolysin, said altered pneumolysin being non-toxic and being capable of eliciting an immune response in an animal being reactive to wild-type pneumolysin.

Preferably the vaccine comprises capsular polysaccharide material conjugated with the altered or mutant pneumolysin.

The capsular material may be derived from any one or more of the *Streptococcus pneumoniae* serotypes 6A, 6B, 14, 18C, 19A, 19F, 23F, 1, 2, 3, 4, 5, 7F, 8, 9N, 9V, 10A, 11A, 12F, 15B, 17F, 20, 22F and 33F.

In this embodiment serotypes which are commonly associated with disease in children, and to which children generally have a poor immune response, may be specifically targeted (i.e. Danish serotypes 6A, 68, 14, 18C, 19A, 19F and 23F). Other common serotypes contained in the present 23-valent Merck Sharp and Dohme vaccine (Pneumovax 23) however, could also be used to synthesize conjugates (i.e. types 1, 2, 3, 4, 5, 7F, 8, 9N, 9V, 10A, 11A, 12F, 15B, 17F, 20, 22F and 33F) or indeed any other serotype. Conjugation of any pneumococcal polysaccharides to the protein carrier ensures good T-cell dependent immunogenicity in children, such that protective levels of anti-polysaccharide antibody are produced.

The combination of the altered pneumolysin together with the capsular material will ensure an extra degree of protection, particularly against serotypes of *S. pneumoniae* whose polysaccharides are not incorporated in the existing vaccine formulations.

The vaccine is preferably administered by sub-cutaneous injection, with or without an approved adjuvant, such as alumina gel.

In another form the invention could be said to reside in a recombinant clone including a replicon and a DNA sequence encoding an altered pneumolysin, said altered pneumolysin being non-toxic and being capable of eliciting an immune response in an animal being reactive to wild-type pneumolysin.

In yet another form the invention could be said to reside in a method of producing an altered pneumolysin including the steps of purifying said altered pneumolysin from an expression system including a recombinant clone with DNA encoding an altered pneumolysin said pneumolysin being substantially non-toxic and being capable of eliciting an immune response in an animal reactive to wild-type pneumolysin.

Preferrably the expression system is a culture of a host cell including a recombinant Clone with DNA encoding the altered pneumolysin.

In another form the invention could be said to reside in a method of producing a vaccine including the step of amplifying a recombinant clone encoding an altered pneumolysin, inducing transcription and translation of said cloned material, the purification of altered pneumolysin, and the step of conjugating the altered pneumolysin with a capsular polysaccharide, the altered pneumolysin having substantially reduced toxic activity as compared with wild-type pneumolysin.

For a better understanding of the invention specific embodiments of the invention will now be described with reference to diagrams wherein:-
FIG. 1 Is the DNA sequence of the gene encoding wild-type pneumolysin,
FIG. 2 Is the DNA sequence of an altered gene encoding wild type pneumoltsin used for cloning the pneumolysin gene into an expression vector,
FIG. 3 Is the amino acid sequence of the wild-type pneumolysin as derived from the DNA sequence of the gene encoding the wild type pneumolysin, and
FIG. 4 shows the amino acid sequence of pneumolysin showing amino acid substitutions introduced by site directed mutagenesis.

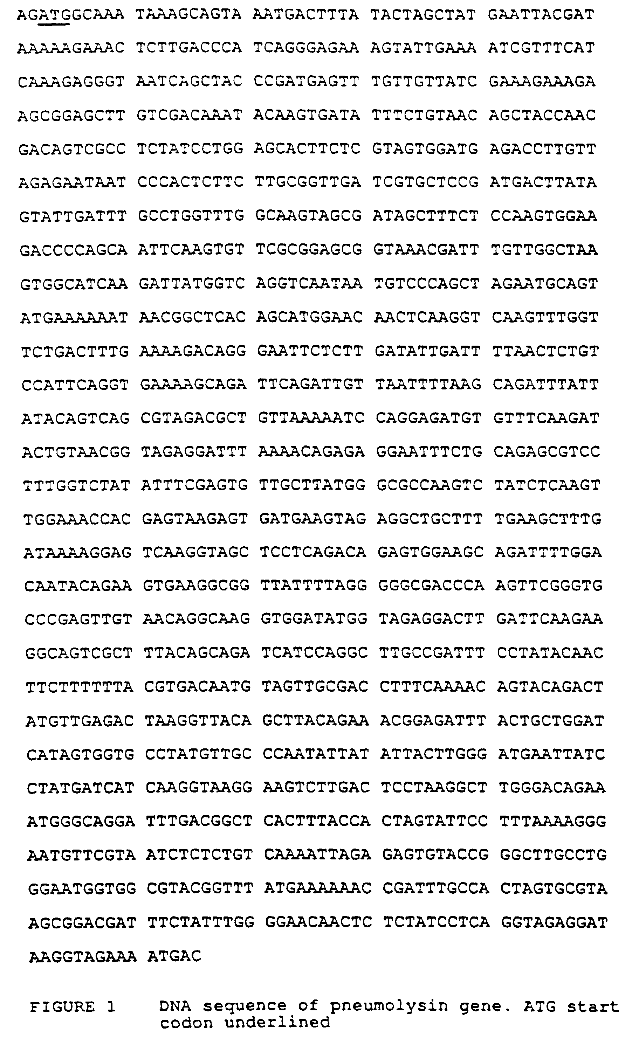

Recombinant DNA techniques have been used to construct non-toxic pneumolysin derivatives suitable for administration to humans. To achieve this, the *S. pneumoniae* gene encoding pneumolysin was cloned into *Escherichia coli* and its complete DNA sequence determined. The DNA sequence is shown in Figure 1 and the derived amino acid sequence is shown in Figure 3.

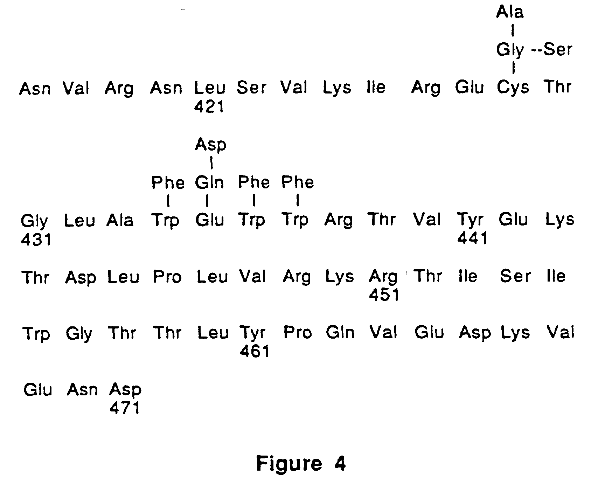

Three regions of the pneumolysin gene were subjected to oligonucleotide-directed mutagenesis. The first region encodes amino acids 427 - 437 in the protein sequence, and is indicated by an underline in Figure 3. This 11 amino acid sequence shows absolute homology with similar regions in other related thiol activated toxins thus is thought to be responsible for the haemolytic acitivity and hence toxic activity of the toxin. The other two regions encode amino acids 257 - 297 and amino acids 368 - 397 and are also indicated by an underline in Figure 3. These two regions of the toxin have substantial amino acid sequence homology with human C-reactive protein (CRP), and by inference therefore, are thought to be responsible for the ability of pneumolysin to bind the Fc region of immunoglobulins and to activate complement. Fifteen separate mutations in the pneumolysin gene, resulting in single amino acid substitutions, were constructed, as shown in Figure 4. In an effort to maintain the structure of the altered pneumolysin, conservative substitutions were made, so that amino acids are substituted with amino acids of a similar nature.

For the region involved in haemolytic activity, Cys ₄₂₈ -> Gly, Cys ₄₂₈ -> Ser, Trp₄₃₃ -> Phe, Glu₄₃₄ -> Asp and Trp₄₃₅ -> Phe each reduced haemolytic activity by 97%, 90%, 99%, 75% and 90% respectively. The other mutations in that region (Cys₄₂₈ -> Ala, Glu₄₃₄ -> Gln and Trp ₄₃₆ ->Phe) did not affect haemolytic activity. Mutating a separate region of the toxin thought to be responsible for binding to target cell membranes also affects haemolytic activity of the protein. This substitution, His₃₆₇ -> Arg, completely inhibits haemolytic activity. This is a quite unpredictable finding in that His₃₆₇ -> Arg therefore shows a greater inhibition of this property than the substitutions made within the 11 amino acid region thought to be responsible for haemolytic activity.

Mutations in the CRP-like domains were tested for ability to activate complement. For Trp₃₇₉ -> Phe, Tyr₃₈₄-> Phe, Asp₃₈₅ -> Asn, and Trp₃₉₇ -> Phe, complement activation was reduced by 20%, 70%, 100% and 15%, respectively. The other mutations in the CRP-like domains shown in Figure 4 do not reduce complement activation. Importantly, the above mutations which affect either haemolytic activity or complement activation do not impair the immunogenicity of the proteins, compared with native or wild-type pneumolysin.

Thus although His₃₆₇ -> Arg is the preferred mutation to reduce the haemolytic activity, a combination of two or more mutants effecting reduced haemolytic activity can also achieve a very high level of reduction in haemolytic activity. Similarly Asp₃₈₅ -> Asn is the prefered mutation to achieve reduced complement activation, however a combination of two or more other mutants that reduce the activity to a lesser degree can also be used.

In a preferred embodiment the pneumolysin derivative for use in the vaccine would contain a combination of certain of the above mutations such that the protein is unable to activate complement in addition to having zero haemolytic activity. Examples of such combination are:-
1) His₃₆₇ -> Arg + Asp₃₈₅-> Asn,
2) His ₃₆₇ -> Arg + Asp₃₈₅ -> Asn + either Cys₄₂₈ -> Gly or Trp₄₃₃ -> Phe
3) Asp₃₈₅ -> Asn + Cys₄₂₈ -> Gly + Trp₄₃₃ -> Phe

These then are some preferred combinations, however it is to be understood that other combinations of mutations can be used to make up the altered pneumolysin for use in a vaccine. Further the altered 5 pneumolysin may comprise any one of the individual mutations with sufficiently reduced activity.

High level expression of the altered pneumolysin from DNA encoding the altered pneumolysin can be achieved by using any one of a number 0 of conventional techniques including the expression in a prokaryotic host with the DNA cloned appropriately within any one of the many expression vectors currently available, or cloned appropriately within the host chromosome; expression in a eukaryotic host with the DNA cloned appropriately either within an expression vector or cloned within the host chromosome; or within an *in vitro* expression system such as may comprise purified components necessary for expression of altered pneumolysin.

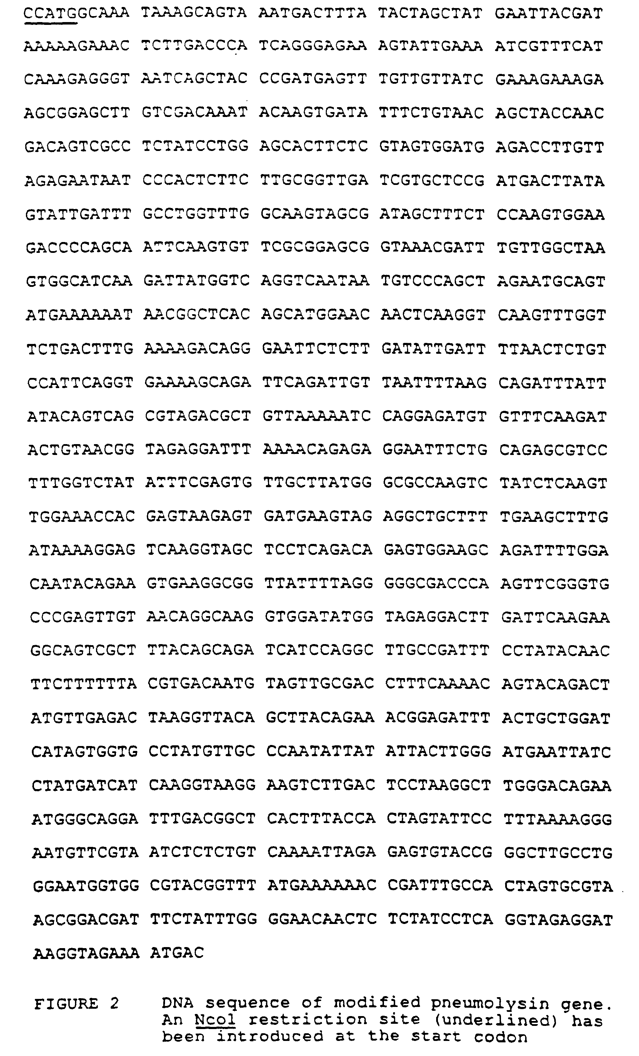

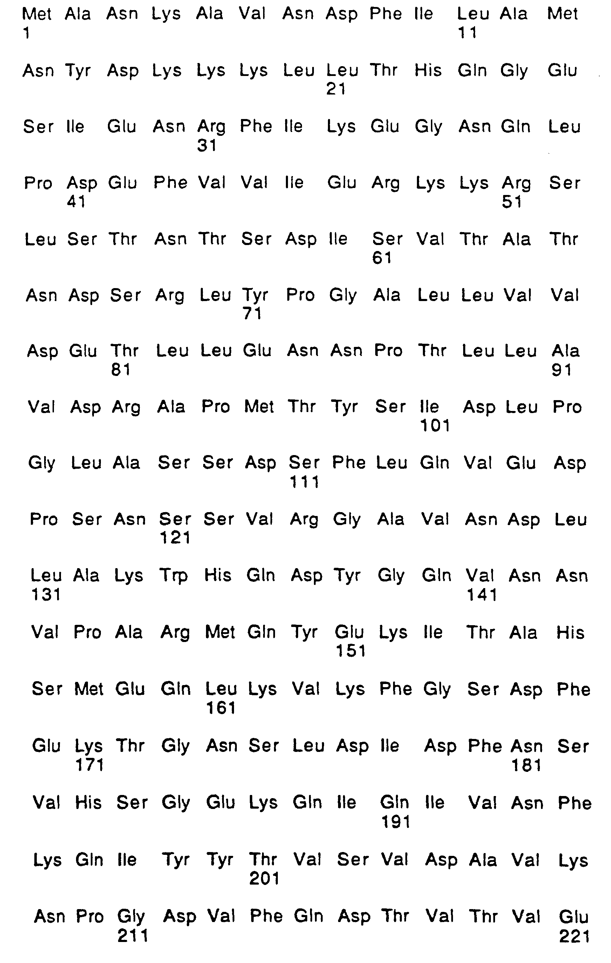

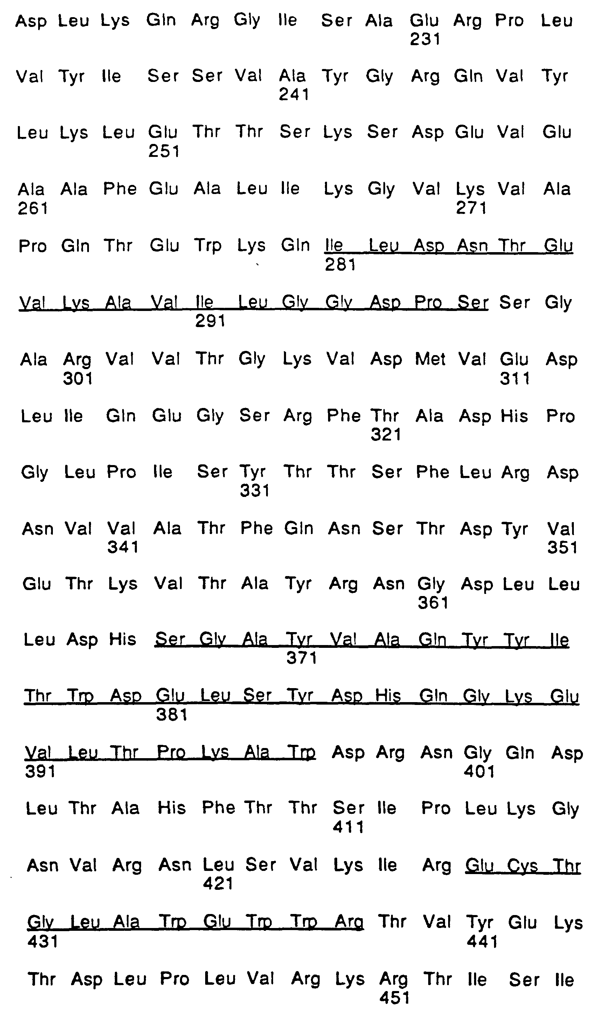

To achieve high level expression of the mutated pneumolysin gene, it has been cloned into the vector pKK233-2 for expression within *Escherichia coli* or other like prokaryote. This vector included ampicillin and tetracycline resistance genes, the *trc* promoter (which can be regulated by IPTG [isopropyl-β-D-thiogalactopyranosidel), and a *lac Z* ribosome binding site adjacent to an ATG initiation codon incorporating an *Nco*I restriction site. Immediately downstream from the initiation codon there are restriction sites for *Pst*I and *Hin*dIII, followed by a strong T₁ T₂ transcription terminator. Prior to insertion into pKK233-2, a *Nco*I restriction site was constructed at the 5' end of the pneumolysin coding sequence (at the initiation codon) by oligonucleotide-directed mutagenesis, as shown in Figure 2. This enabled the proximal end of the altered pneumolysin gene to be cloned into the *Nco*I site of pKK233-2; a *Hin*dIII site approximately 80 bases downstream from the pneumolysin termination codon was used to splice the distal end of the altered gene into the compatible site in pKK233-2. The mutant pneumolysin derivative could however, be cloned into any one of a number of high expression vector systems.

The mutant pneumolysin is prepared as follows: *E. coli* cells harbouring the above recombinant plasmid are first grown in 9 litre cultures in Luria Bertani (or any other appropriate) medium, supplemented with the appropriate antibiotic, at 37° C, with aeration. When the culture reaches the late logarithmic phase of growth, IPTG is added to a final concentration of 20µM (to induce expression of the altered pneumolysin gene) and incubation is continued for a further 2 to 3 hours.

Cells are then harvested by centrifugation or ultrafiltration and lysed by treatment with EDTA and lysozyme, followed by sonication, or by disruption in a French pressure cell. Cell debris is removed by centrifugation and the extract is then dialysed extensively against 10mM sodium phosphate (pH7.0). The material is then loaded onto a column of DEAE-cellulose and eluted with a linear gradient of 10-250mM sodium phosphate (pH7.0). Fractions containing peak levels of the pneumolysin derivative are pooled, concentrated by ultrafiltration and loaded onto a column of Sephacryl S-200. This column is developed in 50mM sodium phosphate (pH7.0) and again fractions with high levels of pneumolysin derivative are pooled, concentrated by ultrafiltration and stored in 50% glycerol at -15°C. The final product is greater than 95% pure, as judged by SDS-polyacrylamide gel electrophoresis. Hydrophobic interaction chromatography on Phenyl-Sepharose is an alternative purification which could also be used. However it is to be understood that this is only one method of purification of the altered pneumolysin, and other, alternative methods (including High Pressure Liquid Chromatography) may be employed.

This purified altered pneumolysin can then be administered as a vaccine at appropriate levels, either by itself or in combination with other antigens. In one form the pneumolysin may be conjugated with polysaccharide derived from any one or more of the variety of pneumococcal strains described above.

The mutant pneumolysin can be conjugated to the various serotypes of polysaccharide by a range of methods. The first involves preparation of an activated polysaccharide by treating pure polysaccharide (available commercially) with cyanogen-bromide and adipicacid dihydrzide (ADH). The ADH-polysaccharide is then combined with the mutant pneumolysin in the presence of 1-ethyl-3-(3-dimethylaminopropyl) carbodiimide - HCI. Conjugated material is separated from the reactants by chromatography through Sepharose CL-4B.

Alternatively, the polysaccharide-mutant pneumolysin conjugates can be prepared using bifunctional reagents such as N-succinimidyl-6(4'-azido-2'-nitrophenylamino)hexanoate (SANPAH). Pure polysaccharide dissolved in phosphate buffered saline, is reacted with SANPAH in the presence of a strong white light source. Unreacted SANPAH is then separated from activated polysaccharide by chromatography on Sephadex G-50. Activated polysaccharide is then conjugated to the mutant pneumolysin in 0.2M borate buffer (pH8.5). Any excess reactive groups are then blocked with lysine, and the polysaccharide-protein conjugate is separated from the other reactants by chromatography on Sepharose CL-4B. Conjugates could also be prepared by reductive amination with cyanoborohydride.

Alternatively another protein, such as inactivated tetanus toxin, can be conjugated with the desired polysaccharides and altered pneumolysin can be added to the vaccine in an unconjugated form.

## Claims

1. A mutant pneumolysin being substantially non-toxic and being capable of eliciting a protective immune response in an animal being reactive to wild-type pneumolysin, **characterized in that** the mutant pneumolysin has the amino acid sequence illustrated in Figure 3, which sequence has been altered by at least one amino acid substitution, deletion or blocking in positions 257 to 297 and/or positions 367 to 397 and/or positions 424-437.

2. A mutant pneumolysin as in Claim 1 which has been altered in positions 367-397 and has reduced complement binding activity as compared to wild-type pneumolysin.

3. A mutant pneumolysin as in Claims 1 or 2 which has been altered in positions 257-297 and has reduced Fc binding activity as compared to wild-type pneumolysin.

4. An altered pneumolysin as in any one of Claims 1-3 having the following amino acid sequence: 
Met Ala Asn Lys Ala Val Asn Asp Phe Ile Leu Ala Met
1 11
Asn Tyr Asp Lys Lys Lys Leu Leu Thr His Gln Gly Glu
21
Ser Ile Glu Asn Arg Phe Ile Lys Glu Gly Asn Gln Leu
31
Pro Asp Glu Phe Val Val Ile Glu Arg Lys Lys Arg Ser
41 51
Leu Ser Thr Asn Thr Ser Asp Ile Ser Val Thr Ala Thr
61
Asn Asp Ser Arg Leu Tyr Pro Gly Ala Leu Leu Val Val
71
Asp Glu Thr Leu Leu Glu Asn Asn Pro Thr Leu Leu Ala
81 91
Val Asp Arg Ala Pro Met Thr Tyr Ser Ile Asp Leu Pro
101
Gly Leu Ala Ser Ser Asp Ser Phe Leu Gln Val Glu Asp
111
Pro Ser Asn Ser Ser Val Arg Gly Ala Val Asn Asp Leu
121
Leu Ala Lys Trp His Gln Asp Tyr Gly Gln Val Asn Asn
131 141
Val Pro Ala Arg Met Gln Tyr Glu Lys Ile Thr Ala His
151
Ser Met Glu Gln Leu Lys Val Lys Phe Gly Ser Asp Phe
161
Glu Lys Thr Gly Asn Ser Leu Asp Ile Asp Phe Asn Ser
171 181
Val His Ser Gly Glu Lys Gln Ile Gln Ile Val Asn Phe
191
 
Lys Gln Ile Tyr Tyr Thr Val Ser Val Asp Ala Val Lys
201
Asn Pro Gly Asp Val Phe Gln Asp Thr Val Thr Val Glu
211 221
Asp Leu Lys Gln Arg Gly Ile Ser Ala Glu Arg Pro Leu
231
Val Tyr Ile Ser Ser Val Ala Tyr Gly Arg Gln Val Tyr
241
Leu Lys Leu Glu Thr Thr Ser Lys Ser Asp Glu Val Glu
251
Ala Ala Phe Glu Ala Leu Ile Lys Gly Val Lys Val Ala
261 271
Pro Gln Thr Glu Trp Lys Gln Ile Leu Asp Asn Thr Glu
281
Val Lys Ala Val Ile Leu Gly Gly Asp Pro Ser Ser Gly
291
Ala Arg Val Val Thr Gly Lys Val Asp Met Val Glu Asp
301 311
Leu Ile Gln Glu Gly Ser Arg Phe Thr Ala Asp His Pro
321
Gly Leu Pro Ile Ser Tyr Thr Thr Ser Phe Leu Arg Asp
331
Asn Val Val Ala Thr Phe Gln Asn Ser Thr Asp Tyr Val
341 351
Glu Thr Lys Val Thr Ala Tyr Arg Asn Gly Asp Leu Leu
361
Leu Asp R1 Ser Gly Ala Tyr Val Ala Gln Tyr Tyr Ile
371
Thr R2 Asp Glu Leu Ser R3 R4 His Gln Gly Lys Glu
381
Val Leu Thr Pro Lys Ala R5 Asp Arg Asn Gly Gln Asp
391 401
Leu Thr Ala His Phe Thr Thr Ser Ile Pro Leu Lys Gly
411
Asn Val Arg Asn Leu Ser Val Lys Ile Arg Glu R6 Thr
421
Gly Leu Ala R7 R8 R9 Trp Arg Thr Val Tyr Glu Lys
431 441
Thr Asp Leu Pro Leu Val Arg Lys Arg Thr Ile Ser Ile
451
Trp Gly Thr Thr Leu Tyr Pro Gln Val Glu Asp Lys Val
461
Glu Asn Asp
471
 wherein R₁ is His or Arg, R₂ is Trp or Phe, R₃ is Tyr or Phe, R₄ is Asp or Asn, R₅ is Trp or Phe, R₆ is Cys, Gly, or Ser, R₇ is Trp or Phe, R₈ is Glu, or Asp, R₉ is Trp or Phe, and wherein at least one of the residues R₁, R₆, R₇, R₈, or R₉ is other than wild-type.

5. An altered pneumolysin as in Claim 4 wherein R₁ is Arg, R₂ is Trp, R₃ is Tyr, R₄ is Asn, R₅ is Trp, R₆ is Cys, R₇ is Trp, R₈ is Glu, and R₉ is Trp.

6. A vaccine comprising an altered pneumolysin as in any one of claims 1 to 5.

7. A vaccine as in Claim 6 comprising capsular polysaccharide material conjugated with a protein carrier and non-conjugated protein material, the capsular polysaccharide material being derived from any one or more than one of the *Streptococcus pneumoniae* serotypes, and the non-conjugated protein material being an altered pneumolysin.

8. A vaccine as in claim 7 wherein the capsular material is derived from any one or more of the *Streptococcus pneumoniae* serotypes 6A, 6B, 14, 18C, 19A, 19F, 23F, 1,2,3,4,5, 7F, 8, 9N, 9V, 10A, 11A, 12F, 15B, 17F, 20, 22F and 33F.

9. A vaccine as in claim 6 comprising capsular polysaccharide material conjugated with a protein carrier, the capsular polysaccharide material being derived from any one or more than one of the *Streptococcus pneumoniae* serotypes, and the protein carrier being an altered pneumolysin.

10. A vaccine as in claim 9 wherein the capsular material is derived from any one or more of the *Streptococcus pneumoniae* serotypes 6A, 6B, 14, 18C, 19A, 19F, 23F, 1,2,3,4,5, 7F, 8, 9N, 9V, 10A, 11A, 12F, 15B, 17F, 20, 22F and 33F.

11. A recombinant plasmid including a DNA sequence encoding an altered pneumolysin as claimed in any one of claims 1 to 5.

12. A hybrid host cell including a recombinant plasmid as claimed in claim 11, said recombinant plasmid including an inducible expression control operable for expression of said altered pneumolysin encoding DNA within a host cell.

13. A method of producing an altered pneumolysin as claimed in any one of claims 1 to 5 including the steps of purifying said altered pneumolysin from an expression system including a recombinant plasmid according to claim 11.

14. A method of producing an altered pneumolysin as claimed in any one of claims 1 to 5 including the steps of purifying said altered pneumolysin from a culture of a host cell according to claim 12.

15. A method of producing a vaccine including the step of amplifying a recombinant clone encoding an altered pneumolysin as claimed in any one of claims 1 to 5, inducing transcription and translation of said cloned material, the purification of altered pneumolysin, and the step of conjugating the altered pneumolysin with a capsular polysaccharide.

## Patentansprüche

1. Mutantes Pneumolysin das im wesentlichen nichttoxisch ist und das eine Immunschutzantwort in einem Tier, das auf Pneumolysin vom Wildtyp reagiert, hervorrufen kann, **dadurch gekennzeichnet, daß** das mutante Pneumolysin die in Figur 3 dargestellte Aminosäuresequenz besitzt, wobei die Sequenz durch mindestens eine Aminosäuresubstitution, -deletion oder -blockierung in Positionen 257 bis 297 und/oder Positionen 367 bis 397 und/oder Positionen 424 bis 437 verändert ist.

2. Mutantes Pneumolysin gemäß Anspruch 1, das in Positionen 367 bis 397 verändert ist und reduzierte komplementäre Bindungsaktivität verglichen mit Pneumolysin vom Wildtyp besitzt.

3. Mutantes Pneumolysin gemäß Anspruch 1 oder 2, das in Positionen 257 bis 297 verändert ist und reduzierte Fc-Bindungsaktivität verglichen mit Pneumolysin vom Wildtyp besitzt.

4. Verändertes Pneumolysin gemäß mindestens einem der Ansprüche 1 bis 3 mit der folgenden Aminosäuresequenz: 
Met Ala Asn Lys Ala Val Asn Asp Phe Ile Leu Ala Met
1                                       11
Asn Tyr Asp Lys Lys Lys Leu Leu Thr His Gln Gly Glu
                            21
Ser Ile Glu Asn Arg Phe Ile Lys Glu Gly Asn Gln Leu
                31
Pro Asp Glu Phe Val Val Ile Glu Arg Lys Lys Arg Ser
    41                                      51
Leu Ser Thr Asn Thr Ser Asp Ile Ser Val Thr Ala Thr
                                61
 
Asn Asp Ser Arg Leu Tyr Pro Gly Ala Leu Leu Val Val
71
Asp Glu Thr Leu Leu Glu Asn Asn Pro Thr Leu Leu Ala
81 91
Val Asp Arg Ala Pro Met Thr Tyr Ser Ile Asp Leu Pro
101
Gly Leu Ala Ser Ser Asp Ser Phe Leu Gln Val Glu Asp
111
Pro Ser Asn Ser Ser Val Arg Gly Ala Val Asn Asp Leu
121
Leu Ala Lys Trp His Gln Asp Tyr Gly Gln Val Asn Asn
131 141
Val Pro Ala Arg Met Gln Tyr Glu Lys Ile Thr Ala His
151
Ser Met Glu Gln Leu Lys Val Lys Phe Gly Ser Asp Phe
161
Glu Lys Thr Gly Asn Ser Leu Asp Ile Asp Phe Asn Ser
171 181
Val His Ser Gly Glu Lys Gln Ile Gln Ile Val Asn Phe
191
Lys Gln Ile Tyr Tyr Thr Val Ser Val Asp Ala Val Lys
201
Asn Pro Gly Asp Val Phe Gln Asp Thr Val Thr Val Glu
211 221
Asp Leu Lys Gln Arg Gly Ile Ser Ala Glu Arg Pro Leu
231
Val Tyr Ile Ser Ser Val Ala Tyr Gly Arg Gln Val Tyr
241
Leu Lys Leu Glu Thr Thr Ser Lys Ser Asp Glu Val Glu
251
Ala Ala Phe Glu Ala Leu Ile Lys Gly Val Lys Val Ala
261 271
Pro Gln Thr Glu Trp Lys Gln Ile Leu Asp Asn Thr Glu
281
Val Lys Ala Val Ile Leu Gly Gly Asp Pro Ser Ser Gly
291
 
Ala Arg Val Val Thr Gly Lys Val Asp Met Val Glu Asp
301 311
Leu Ile Gln Glu Gly Ser Arg Phe Thr Ala Asp His Pro
321
Gly Leu Pro Ile Ser Tyr Thr Thr Ser Phe Leu Arg Asp
331
Asn Val Val Ala Thr Phe Gln Asn Ser Thr Asp Tyr Val
341 351
Glu Thr Lys Val Thr Ala Tyr Arg Asn Gly Asp Leu Leu
361
Leu Asp R1 Ser Gly Ala Tyr Val Ala Gln Tyr Tyr Ile
371
Thr R2 Asp Glu Leu Ser R3 R4 His Gln Gly Lys Glu
381
Val Leu Thr Pro Lys Ala R5 Asp Arg Asn Gly Gln Asp
391 401
Leu Thr Ala His Phe Thr Thr Ser Ile Pro Leu Lys Gly
411
Asn Val Arg Asn Leu Ser Val Lys Ile Arg Glu R6 Thr
421
Gly Leu Ala R7 R8 R9 Trp Arg Thr Val Tyr Glu Lys
431 441
Thr Asp Leu Pro Leu Val Arg Lys Arg Thr Ile Ser Ile
451
Trp Gly Thr Thr Leu Tyr Pro Gln Val Glu Asp Lys Val
461
Glu Asn Asp
471
 worin bedeuten: R₁ His oder Arg, R₂ Trp oder Phe, R₃ Tyr oder Phe, R₄ Asp oder Asn, R₅ Trp oder Phe, R₆ Cys, Gly oder Ser, R₇ Trp oder Phe, R₈ Glu oder Asp, R₉ Trp oder Phe, und worin mindestens einer der Reste R₁, R₆, R₇, R₈ oder R₉ anders als vom Wildtyp ist.

5. Verändertes Pneumolysin gemäß Anspruch 4, worin bedeuten: R₁ Arg, R₂ Trp, R₃ Tyr, R₄ Asn, R₅ Trp, R₆ Cys, R₇ Trp, R₈ Glu und R₉ Trp.

6. Impfstoff umfassend ein verändertes Pneumolysin gemäß mindestens einem der Ansprüche 1 bis 5.

7. Impfstoff gemäß Anspruch 6, umfassend Kapselpolysaccharid-Material, das mit einem Proteinträger konjugiert ist und nicht-konjugiertes Proteinmaterial, wobei das Kapselpolysaccharid-Material aus einem oder mehr als einem der Streptococcus-Pneumoniae-Serotypen stammt, und das nicht-konjugierte Proteinmaterial verändertes Pneumolysin ist.

8. Impfstoff gemäß Anspruch 7, worin das Kapselmaterial erhältlich ist aus einem oder mehr als einem der Streptococcus-Pneumoniae-Serotypen 6A, 6B, 14, 18C, 19A, 19F, 23F, 1, 2, 3, 4, 5, 7F, 8, 9N, 9V, 10A, 11A, 12F, 15B, 17F, 20, 22F und 33 F.

9. Impfstoff gemäß Anspruch 6, umfassend Kapselpolysaccharid-Material, das mit einem Proteinträger konjugiert ist, wobei das Kapselpolysaccharid-Material aus einem oder mehr als einem der Streptococcus-Pneumoniae-Sterotypen erhältlich ist, und der Proteinträger ein verändertes Pneumolysin ist.

10. Impfstoff gemäß Anspruch 9, worin das Kapselmaterial erhältlich ist aus einem oder mehr als einem der Streptococcus-Pneumoniae-Serotypen 6A, 6B, 14, 18C, 19A, 19F, 23F, 1, 2, 3, 4, 5, 7F, 8, 9N, 9V, 10A, 11A, 12F, 15B, 17F, 20, 22F und 33 F.

11. Rekombinantes Plasmid einschließlich einer DNA-Sequenz, die ein verändertes Pneumolysin gemäß mindestens einem der Ansprüche 1 bis 5 kodiert.

12. Hybride Wirtszelle einschließlich einem rekombinanten Plasmid gemäß Anspruch 11, wobei das rekombinante Plasmid eine induzierbares Expressionskontrolle einschließt, die zum Exprimieren des veränderten Pneumolysins, das DNA in einer Wirtszelle kodiert, wirksam ist.

13. Verfahren zur Herstellung eines verändertes Pneumolysins gemäß mindestens einem der Ansprüche 1 bis 5, das die Reinigungsschritte, des veränderten Pneumolysins aus einem Expressionssystem einschließlich eines rekombinanten Plasmids gemäß Anspruch 11 einschließt.

14. Verfahren zur Herstellung eines veränderten Pneumolysins gemäß mindestens einem der Ansprüche 1 bis 5, das die Reinigungsschritte des veränderten Pneumolysins aus einer Kultur einer Wirtszelle gemäß Anspruch 12 einschließt.

15. Verfahren zur Herstellung eines Impfstoffs, das den Schritt des Amplifizierens eines rekombinanten Klons, das ein verändertes Pneumolysin gemäß mindestens einem der Ansprüche 1 bis 5 kodiert, das die Transkription und Translation des klonierten Materials, die Reinigung von verändertem Pneumolysin induziert, und den Schritt des Konjugierens des geänderten Pneumolysins mit einem Kapselpolysaccharid einschließt.

## Revendications

1. Pneumolysine mutante, essentiellement non-toxique et capable de déclencher une réponse immune protectrice chez un animal réactif vis-à-vis de la pneumolysine de type sauvage, **caractérisée en ce que** la pneumolysine mutante a la séquence d'acides aminés illustrée sur la Figure 3, laquelle séquence a été altérée par au moins une substitution, une délétion ou un blocage d'acides aminés sur les positions 257 à 297 et/ou sur les positions 367 à 397 et/ou sur les positions 424 à 437.

2. Pneumolysine mutante selon la revendication 1, qui a été altérée sur les positions 367 à 397 et présente une activité réduite de liaison au complément par comparaison à la pneumolysine de type sauvage.

3. Pneumolysine mutante selon les revendications 1 ou 2, qui a été altérée sur les positions 257 à 297 et a une activité réduite de liaison du Fc par comparaison à la pneumolysine de type sauvage.

4. Pneumolysine altérée selon l'une quelconque des revendications 1 à 3, ayant la séquence d'acides aminés suivante : 
Met Ala Asn Lys Ala Val Asn Asp Phe Ile Leu Ala Met
1 11
Asn Tyr Asp Lys Lys Lys Leu Leu Thr His Gln Gly Glu
21
Ser Ile Glu Asn Arg Phe Ile Lys Glu Gly Asn Gln Leu
31
Pro Asp Glu Phe Val Val Ile Glu Arg Lys Lys Arg Ser
41 51
Leu Ser Thr Asn Thr Ser Asp Ile Ser Val Thr Ala Thr
61
Asn Asp Ser Arg Leu Tyr Pro Gly Ala Leu Leu Val Val
71
Asp Glu Thr Leu Leu Glu Asn Asn Pro Thr Leu Leu Ala
81 91
Val Asp Arg Ala Pro Met Thr Tyr Ser Ile Asp Leu Pro
101
Gly Leu Ala Ser Ser Asp Ser Phe Leu Gln Val Glu Asp
111
Pro Ser Asn Ser Ser Val Arg Gly Ala Val Asn Asp Leu
121
Leu Ala Lys Trp His Gln Asp Tyr Gly Gln Val Asn Asn
131 141
Val Pro Ala Arg Met Gln Tyr Glu Lys Ile Thr Ala His
151
Ser Met Glu Gln Leu Lys Val Lys Phe Gly Ser Asp Phe
161
Glu Lys Thr Gly Asn Ser Leu Asp Ile Asp Phe Asn Ser
171 181
Val His Ser Gly Glu Lys Gln Ile Gln Ile Val Asn Phe
191
Lys Gln Ile Tyr Tyr Thr Val Ser Val Asp Ala Val Lys
201
Asn Pro Gly Asp Val Phe Gln Asp Thr Val Thr Val Glu
211 221
Asp Leu Lys Gln Arg Gly Ile Ser Ala Glu Arg Pro Leu
231
Val Tyr Ile Ser Ser Val Ala Tyr Gly Arg Gln Val Tyr
241
Leu Lys Leu Glu Thr Thr Ser Lys Ser Asp Glu Val Glu
251
Ala Ala Phe Glu Ala Leu Ile Lys Gly Val Lys Val Ala
261 271
Pro Gln Thr Glu Trp Lys Gln Ile Leu Asp Asn Thr Glu
281
Val Lys Ala Val Ile Leu Gly Gly Asp Pro Ser Ser Gly
291
Ala Arg Val Val Thr Gly Lys Val Asp Met Val Glu Asp
301 311
Leu Ile Gln Glu Gly Ser Arg Phe Thr Ala Asp His Pro
321
 
Gly Leu Pro Ile Ser Tyr Thr Thr Ser Phe Leu Arg Asp
331
Asn Val Val Ala Thr Phe Gln Asn Ser Thr Asp Tyr Val
341 351
Glu Thr Lys Val Thr Ala Tyr Arg Asn Gly Asp Leu Leu
361
Leu Asp R1 Ser Gly Ala Tyr Val Ala Gln Tyr Tyr Ile
371
Thr R2 Asp Glu Leu Ser R3 R4 His Gln Gly Lys Glu
381
Val Leu Thr Pro Lys Ala R5 Asp Arg Asn Gly Gln Asp
391 401
Leu Thr Ala His Phe Thr Thr Ser Ile Pro Leu Lys Gly
411
Asn Val Arg Asn Leu Ser Val Lys Ile Arg Glu R6 Thr
421
Gly Leu Ala R7 R8 R9 Trp Arg Thr Val Tyr Glu Lys
431 441
Thr Asp Leu Pro Leu Val Arg Lys Arg Thr Ile Ser Ile
451
Trp Gly Thr Thr Leu Tyr Pro Gln Val Glu Asp Lys Val
461
Glu Asn Asp
471
 où R₁ est His ou Arg, R₂ est Trp ou Phe, R₃ est Tyr ou Phe, R₄ est Asp ou Asn, R₅ est Trp ou Phe, R₆ est Cys, Gly ou Ser, R₇ est Trp ou Phe, R₈ est Glu ou Asp, R₉ est Trp ou Phe, et où au moins l'un des résidus R₁, R₆, R₇, R₈ ou R₉ est autre qu'un résidu de type sauvage.

5. Pneumolysine altérée selon la revendication 4, dans laquelle R₁ est Arg, R₂ est Trp, R₃ est Tyr, R₄ est Asn, R₅ est Trp, R₆ est Cys, R₇ est Trp, R₈ est Glu et R₉ est Trp.

6. Vaccin comprenant une pneumolysine altérée selon l'une quelconque des revendications 1 à 5.

7. Vaccin selon la revendication 6, comprenant un matériau polysaccharidique capsulaire conjugué à un support protéique et un matériau protéique non-conjugué, le matériau polysaccharidique capsulaire dérivant d'un ou plusieurs quelconques des sérotypes de ***Streptococcus pneumoniae*,** et le matériau protéique non-conjugué étant une pneumolysine altérée.

8. Vaccin selon la revendication 7, dans lequel le matériau capsulaire dérivé d'un ou plusieurs quelconques des sérotypes de ***Streptococcus pneumoniae*** 6A, 6B, 14, 18C, 19A, 19F, 23F, 1,2,3,4,5, 7F, 8, 9N, 9V, 10A, 11A, 12F, 15B, 17F, 20, 22F et 33F.

9. Vaccin selon la revendication 6, comprenant un matériau polysaccharidique capsulaire conjugué à un support protéique, le matériau polysaccharidique capsulaire dérivant d'un ou plusieurs quelconques des sérotypes de ***Streptococcus pneumoniae***, et le support protéique étant une pneumolysine altérée.

10. Vaccin selon la revendication 9, dans lequel le matériau capsulaire dérivé d'un ou plusieurs quelconques des sérotypes de ***Streptococcus pneumoniae*** 6A, 6B, 14, 18C, 19A, 19F, 23F, 1,2,3,4,5, 7F, 8, 9N, 9V, 10A, 11A, 12F, 15B, 17F, 20, 22F et 33F.

11. Plasmide recombinant comprenant une séquence d'ADN codant pour une pneumolysine altérée selon l'une quelconque des revendications 1 à 5.

12. Cellule hôte hybride comprenant un plasmide recombinant selon la revendication 11, ledit plasmide recombinant comprenant un témoin d'expression inductible pouvant être utilisé pour l'expression dudit ADN codant pour la pneumolysine altérée à l'intérieur d'une cellule hôte.

13. Procédé de production d'une pneumolysine altérée selon l'une quelconque des revendications 1 à 5, qui comprend les étapes de purification de ladite pneumolysine altérée à partir d'un système d'expression comprenant un plasmide recombinant selon la revendication 11.

14. Procédé de production d'une pneumolysine altérée selon l'une quelconque des revendications 1 à 5, qui comprend les étapes de purification de ladite pneumolysine altérée à partir d'une culture d'une cellule hôte selon la revendication 12.

15. Procédé de production d'un vaccin, qui comprend l'étape d'amplification d'un clone recombinant codant pour une pneumolysine altérée selon l'une quelconque des revendications 1 à 5, l'induction de la transcription et de la traduction dudit matériau cloné, la purification de la pneumolysine altérée, et l'étape de conjugaison de la pneumolysine altérée à un polysaccharide capsulaire.
